(19) Europäisches Patentamt
European Patent Office
Office européen des brevets

(11) Veröffentlichungsnummer: **0 069 692**
**B1**

(12) **EUROPÄISCHE PATENTSCHRIFT**

(45) Veröffentlichungstag der Patentschrift: **02.03.88**

(21) Anmeldenummer: **82730069.0**

(22) Anmeldetag: **19.05.82**

(51) Int. Cl.[4]: **C 07 C 33/12**, C 07 C 43/178, C 07 D 317/72, C 07 D 309/12, A 61 K 31/045, A 61 K 31/075 // C07C177/00, A61K31/37

(54) **Neue Carbacycline, Verfahren zu ihrer Herstellung und ihre Verwendung als Arzneimittel.**

(30) Priorität: **22.05.81 DE 3121155**

(43) Veröffentlichungstag der Anmeldung: **12.01.83 Patentblatt 83/02**

(45) Bekanntmachung des Hinweises auf die Patenterteilung: **02.03.88 Patentblatt 88/09**

(84) Benannte Vertragsstaaten: **AT BE CH DE FR GB IT LI LU NL SE**

(56) Entgegenhaltungen:
**DE-A-2 902 442**
**DE-A-2 904 655**
**FR-A-2 337 714**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Skuballa, Werner, Dr.**
**Olwenstrasse 23**
**D-1000 Berlin 28 (DE)**
Erfinder: **Radüchel, Bernd, Dr.**
**Gollanczstrasse 132**
**D-1000 Berlin 28 (DE)**
Erfinder: **Vorbrüggen, Helmut, Prof.Dr.**
**Wilkestrasse 7**
**D-1000 Berlin 27 (DE)**
Erfinder: **Casals-Stenzel, Jorge, Dr.**
**Wichernstrasse 20**
**D-1000 Berlin 33 (DE)**
Erfinder: **Schillinger, Ekkehard, Dr.**
**Im Amseltal 50**
**D-1000 Berlin 28 (DE)**
Erfinder: **Mannesmann, Gerda, Dr.**
**Hagenplatz 3e**
**D-1000 Berlin 33 (DE)**
Erfinder: **Nieuweboer, Bob**
**Carstenstrasse 44**
**D-1000 Berlin 45 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 069 692 B1

**Beschreibung**

Die Erfindung betrifft neue Prostacyclin-Derivate, Verfahren zu ihrer Herstellung sowie inhre Verwendung als Arzneimittel.

In den deutschen Offenlegungsschriften DE—OS—29 00 352, 29 02 442, 29 04 655, 29 09 088 und 29 12 409 werden (5E)- und (5Z)-6a-Carbaprostaglandin-$I_2$-Analoga berschrieben. Die Nomenklatur der erfindungsgemäßen Verbindungen basiert auf einem Vorschlag von Morton und Brokow (J. Org. Chem. *44*, 2880[19798]). Bei der Synthese dieser Verbindungen entstehen stets zewi Doppelbindungisomere, die durch den Zusatz (5E) oder (5Z) charakterisiert werden. Die beiden Isomeren dieses Prototyps werden durch folgende Struckturformeln verdeutlicht:

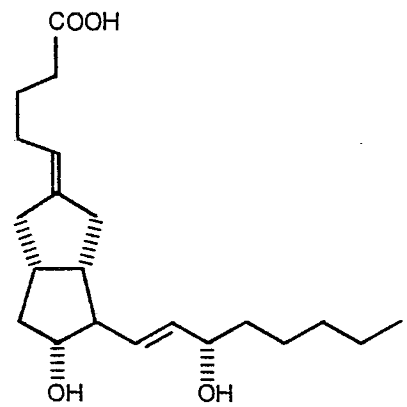

(5E)—6a—Carbaprostaglandin—$I_2$

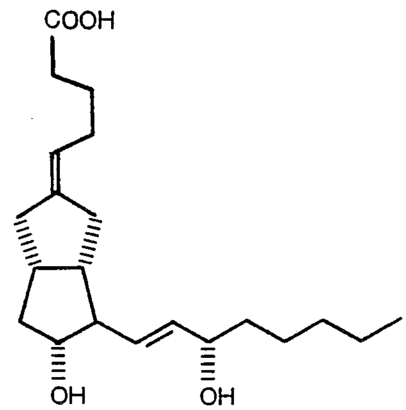

(5Z)—6a—Carbaprostaglandin—$I_2$

Aus dem sehr umfangreichen Stand der Technik der Prostacycline und ihrer Analoga weiß man, daß diese Stoffklasse aufgrund ihrer biologischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

In EP 11 591 werden Carbacycline beschrieben, die durch Einführung eines 6a-Carba-Gruppe anstelle des 9-Äthersauerstoffatoms in PG $7_2$-Molekül stabilisiert wurden und die bei gleichem pharmakologischen Wirkungsspektrum (Bluitdrucksenkung, Bronchiodilatation und Thrombozytenaggregationshemmung) eine verlängerte Wirkungsdauer zeigen.

Es wurde nun gefunden, daß durch Ersatz der 1-Carboxylgruppe in den 6a-Carbacyclinen und 6a-3-Oxa-carbacylinen durch eine Hydroxymethylgruppe eine längere Wirkungsdauer, größere Selektivität und bessere Wirksamkeit erzielt werden können. Die erfindungsgemäßen Verbindungen wirken blutdrucksenkend und bronchodilatatorisch. Sie sind außerdem zur Vasodilatation, der Inhibierung der Thrombozytenaggregation und der Hemmung der Magensäuresekretion geeignet.

Die Erfindung betrifft Carbacyclinderivate der allgemeinen Formel I

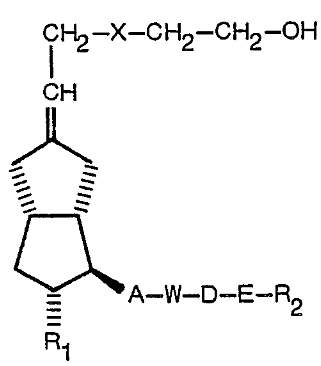

I,

worin

X ein Sauerstoffatom oder eine —$CH_2$-Gruppe,

A eine —$CH_2$—$CH_2$, eine trans-CH=CH— oder eine —C≡C-Gruppe,

W eine freie oder funktionell abgewandelte Hydroxymethylengruppe oder eine freie oder funktionell abgewandelte Hydroxymethylengruppe, wobei die OH-Gruppe α- oder β-ständig sein können,

D eine geradkettige gesättigte Alkylengruppe mit 1—10 C-Atomen oder eine verzweigte gesättigte oder ungesättigte Alkylengruppe mit 2—10 C-Atomen, die durch $C_1$—$C_4$-Alkyl oder Fluoratome substituiert sein können, und Doppelbindungen sich in 2- oder 3-Stellung befinden, wenn D 4—10 C-Atome hat,

E eine —C≡C-Gruppe oder eine —$CR_3$≡$CR_4$-Gruppe, wobei $R_3$ und $R_4$ verschieden sind und ein Wasserstoffatom oder eine Alkylgruppe mit 1—5 C-Atomen bedeuten,

$R_1$ eine freie oder funktionell abgewandelte Hydroxygruppe, gerad -oder verzweigtkettiges, gesättigtes oder ungesättigtes Alkyl mit 1—10.

$R_2$ ein C-Atomen, Phenyl, 1-Naphthyl und 2-Napthyl, durch 1—3 Halogenatome, eine Phenylgruppe, 1—3 Alkylgruppen mit jeweils 1—4 C-Atomen, eine Chlormethyl, Fluormethyl, Triflourmethyl, Carboxyl oder $C_1$—$C_4$ ALkoxy -oder Hydroxygruppe substituiertes Phenyl, 1-Napthyl oder 2-Napthyl, oder eine 5 -oder 6-gliedrigeheterocyclische Gruppe mit einem N—, O -oder S-Atom bedeuten.

Die Hydroxygruppen $R_1$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Terahydrofuranyl-, α-Äthoxyäthyl, Trimethylsilyl-, Dimethyl-tert.-butylsilyl- und Tribenzylsilylrest. Als Acylreste kommen $C_1$—$C_4$-Alkanoylreste wie beispielsweise Acetyl, Propionyl oder Butyryl in Frage.

Als Alkylgruppen $R_2$ kommen gesättigte Alkylreste mit 1—4 C-Atomen, in Frage. Beispielsweise genannt seien Methyl-, Äthyl-, Propyl, Butyl, Isobutyl-, tert.-Butyl-.

Als Alkylengruppe D kommen geradkettige Alkylenreste mit 1—5 C-Atomen in Frage, die gegebenenfalls durch $C_1$—$C_4$-Alkyl in 1- oder 2-Stellung substituiert sein können. Beispielsweise genannt seien: Methylen, Äthylen, 1,2-Propylen, Äthyläthylen, Trimethylen, Tetramethylen, Pentamethylen, 1-Methyltetramethylen, 1-Methyl-trimethylen, 2-Methyl-trimethylen, 2-Methyl-tetramethylen.

Die Alkylgruppen $R_3$, $R_4$ und $R_5$ können gerad- oder verzweigtkettig sein und 1—5 C-Atome haben. Als namentliche Beispiele kommen reste mit bis zu 5 C-Atomen in Betracht, wie sie für die Alkylgruppen $R_2$ bereits gennant worden sind.

Die Erfindung betrifft ferner ein Verfahren zur Herstellung der erfindungsgemäßen Carbacyclinderivate der allgemeinen Formel I, dadurch gekennzeichnet, daß man in an sich bekannter Weise eine Verbindung der algemeinen Formel II

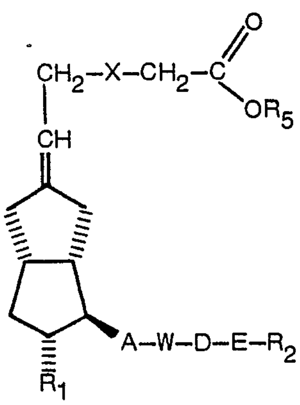

II,

worin

$R_1$, $R_2$, X, A, W, D und E die obenangegebenen Bedeutungen aufweisen und $R_5$ einen Alkylrest mit 1—5 C-Atomen oder ein Wasserstoffatom bedeutet, reduziert und gegebenenfalls anschließend in beliebiger Reihenfolge Isomere trennt und/oder geschützte Hydroxygruppen freisetzt.

Die Reduktion der Verbindungen der allgemeinen Formel II wird mit einem für die Reduktion von Estern oder Carbonsäuren geeigneten Reduktionsmittel wie beispielsweise Lithiumaluminiumhydrid, Diisobutylaluminiumhydrid usw., durchgeführt. Als Lösungsmittel kommen in Frage: Diäthyläther, Tetrahydrofuran, Diäthylenglykoldimethyläther, Toluol usw. Die Reduktion wird bei Temperaturen won −30° bis zur Siedetemperatur des verwendeten Lösungsmittels, vorzugsweise 0°C bis 30°C, vorgenommen.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie z.B. Salzsäure, durchgeführt. Zur Verbesserung der Löslichkeit wird zweckmäßigerweise ein mit Wasser mischbares inertes organisches Lösungsmittel zugestzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beisepielsweise mit Tetrabutylammoniumfluorid oder mit KF in Gengenwart eines Kronenäthers. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die als Ausangsmaterial dienenden Verbindungen der allgemeinen Formel II können beispielsweise hergestellt werden, indem man in an sich bekannter Weise einen Aldehyd der Formel III 'DE—OS 28 45 770)

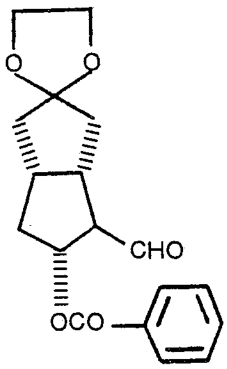

III

mit einem Phosphonat der allgemeinen Formel IV

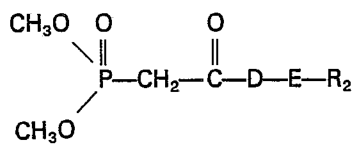

IV

worin D, E und $R_2$ die obenangegebene Bedeutung aufweisen, in einer Olefinierungsreaktion zu einem Keton der allgemeinen Formel V umsetzt.

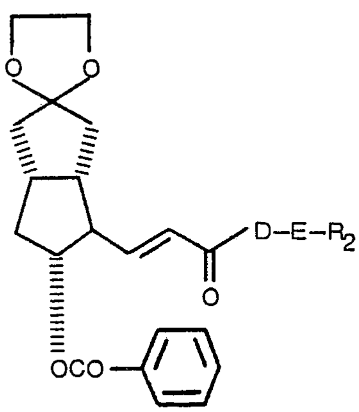

V

Nach Reduktion der Ketogruppe mit Zinkborhydrid oder Natriumborhydrid oder Umsetzung mit Alkylmagnesiumbromid oder Alkyllithium und gegebenfalls anschließender Epimerentrennung, sowie gegebenenfalls Hydrierung der Doppelbindung, gelangt man zu den Verbindungen der allgemienen Formel VI.

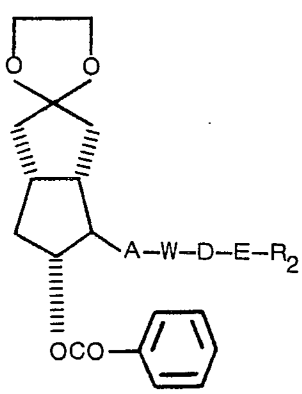

VI

Verseifung der Estergruppe, beispielsweise mit Käliumcarbonat in Methanol und Ketalspaltung mit wässriger Essigsäure sowie gegebenenfalls funktionelle Abwandlung der freien Hydroxygruppen, beispielsweise durch Verätherung mit Dihydropyran liefert das Keton der allgemeinen Formel VII.

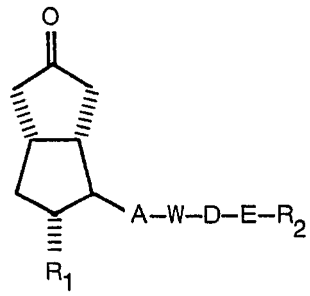

VII

Nach Olefinierungsreaktion mit Phosphonoessigsäuretriäthylester oder Phosphonoessigsäuretrimethylester und anschließender Reduktion mit Lithiumaluminiumhydrid erhält man die an der Doppelbindung isomeren Verbindungen der allgemeinen Formel VIII, die gegebenenfalls getrennt werden können.

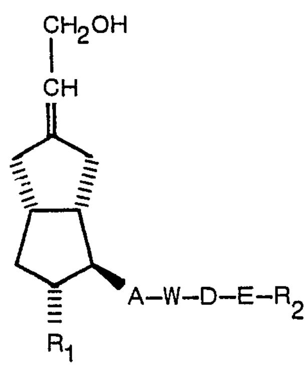

VIII

Die Verätherung des Alkohols VIII mit einem Halongenssigsäurederivat der allgemeinen Formel IX,

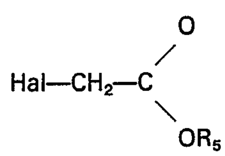

IX

wobei Hal ein Chlor- oder Bromaton und $R_5$ einen Alkylrest mit 1—5 C-Atomen oder ein Wasserstoffatom oder ein Alkalimetall bedeutet, in Gegenwart einer Base und gegebenenfalls anschließende Veresterung liefert die Verbindungen der allgemeinen Formel II mit X in der Bedeutung eines Sauerstoffatoms.

Die Umsetzung der Verbindung der allgemeinen Formel VIII mit einem Halogenessigsäurederivat der allgemeinen Formel IX wird bei Temperaturen von 0°C bis 100°C, vorzugsweise 10°C bis 80°C, in einem aprotischen Lösungsmittel oder Lösungsmittelgemisch, beispielsweise Dimethylsulfoxid, Dimethylformamid, Tetrahydrofuran, Diätheylenglykoldimethyläther usw., vorgenommen. Als Basen kommen die dem Fachmann für Verätherung bekannten Basen in Frage, beispielsweise Natriumhydrid, Kalium-tert.-butylat, Butyllithium usw.

Die als Ausgangsmaterial dienenden Verbindungen der allgemeinen Formel II mit X in der Bedeutung einer $CH_2$-Gruppe können beispielsweise hergestellt werden, indem man in an sich bekannter Weise ein Keton der Formel VII mit einem Wittig-Reagenz der Formel X

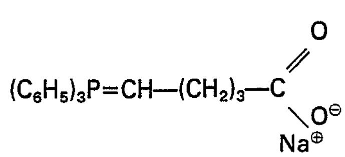

X

umsetzt und gegebenenfalls anschließend die Doppelbindungsisomeren trennt und die freie Carboxylgruppe verestert.

Die Herstellung der Phosphonate der allgemeinen Formel IV erfolgt in an sich bekannter Weise durch

Umsetzung eines Alkylhalogenids (herstellbar aus dem entsprechenden Alkohol durch Halogenierung) der allgemeinen Formel XI

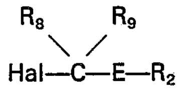

XI

mit dem aus dem Phosphonat der allgemeinen Formel XII erzeugten Dianion,

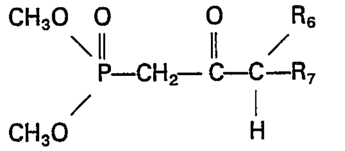

XII

worin $R_6$, $R_7$, $R_8$ und $R_9$ ein Wasserstoffatom, ein Fluoratom oder eine Alkylgruppe mit 1—5 C-Atomen bedeuten können und $R_2$ und E die obenangegebene Bedeutung aufweisen.

Ein weiterer Zugang zu den Phosphonaten der allgemeinen Formel IV besteht in der Umsetzung des Anions von Methylphosphonsäuredimethylester mit einem Ester der allgemeinen Formel XIII,

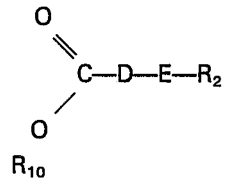

XIII

worin D, E und $R_2$ die obenangegebenen Bedeutungen aufweisen und $R_{10}$ eine Alkylgruppe mit 1—5 C-Atomen bedeutet, den man aus dem entsprechenden Malonsäureester durch Alkylierung mit dem Halogenid der allgemeinen Formel XI und anschließender Decarbalkoxylierung erhalten kann. Der Ester der allgemeinen Formel XIII ist auch aus dem Ester der allgemeinen Formel XIV

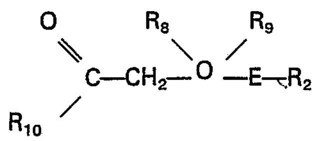

XIV

durch Alkylierung mit dem entsprechenden Alkylhalogenid zugänglich.

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostacyclinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Carbacycline zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ. Die neuen Carbacycline wirken zytoprotektiv an der Leber und Bauchspeicheldrüse und eignen sich zur Prophylaxe und Therapie ischämischer Attacken des ZNS-Systems, Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung. Außerdem besitzen die neuen Carbacycline antiproliferative und antidiarrhoegene (d.h. verhindern Flüssigkeitsansammlungen in Dünndärmen) Eigenschaften und können in Kombination mit ß-Blockern und Diuretika verwendet werden.

Die wirksame Dosis der Verbindungen liegt zwischen 1—1500 µg/kg/Tag, wenn sie am menschlichen Patienten verabreicht werden. Die Einheitsdosis für den pharmazeutischen akzeptablen Träger beträgt 0,01—100 mg.

Bei oraler Applikation an wachen, hypertonen Ratten zeigen die erfindungsgemäßen Verbindungen eine stärkere blutdrucksenkende und länger anhaltende Wirkung als $PGE_2$ und $PGA_2$, ohne wie $PGE_2$Durchfälle oder $PGA_2$ kardiale Arrhythmien auszulösen.

An narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft somit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Beispiel 1

(5E)-(16RS)-2-Descarboxy-2-hydroxymethyl-3-oxa-16-methyl-18,18,19,19-tetradehydro-6a-carba-prostaglandin-$I_2$

Eine Lösung von 360 mg (5E)-(16RS)-16-Methyl-3-oxa-18,18,19,19-tetradehydro-6a-carba-prostaglandin-$I_2$-methylester-11,15-bis-(tetrahydropyranyläther) in 25 ml Tetrahydrofuran versetzt man bei 0°C portionweise mit 180 mg Lithiumaluminiumhydrid und rührt 30 Minuten bei 0°C. Anschließend zerstört man überschüssiges Reagenz durch tropfenweise Zugabe von Essigester, versetzt mit 3 ml Wasser, rührt 1 Stunde, filtriert und dampft im Vakuum ein. Dabei erhält man 350 mg (5E)-(16RS)-2-Descarboxy-2-hydroxymethyl-3-oxa-16-methyl-,18,18,19,19-tetradehydro-6a-carba-prostaglandin-$I_2$-11,15-bis-(tetrahydropyranyläther) als farbloses Öl.

IR ($CHCl_3$): 3600, 3450, 2942, 2870, 1453, 1440, 972/cm.

Zur Abspaltung der Schutzgruppen rührt man 320 mg des Bis-tetrahydropryanylätheres mit 30 ml einer Mischung aus Essigsäure/Wasser/Tetrahydrofuran (65 + 35 + 10) 16 Studen bei Raumtemperatur und dampft anschließend im Vakumm ein. Den Rückstand chromatographiert man mit Methylenchlorid/Isopropanol (9 + 1) an Kieselgel. Dabei erhält man 162 mg der Titelverbindung als farbloses Öl.

IR: 3600, 3430, 2999, 2922, 2860, 1600, 1455, 1430, 1105, 971/cm.

Das Ausgang material für die obige Titelverbindung wird wie folgt hergestellt:

1a) (5E)-(16RS)-16-Methyl-3-oxa-18,18,19,19-tetradehydro-6a-carba-prostaglandin-$I_2$-methylester-11,15-bis-(tetrahydropyranyläther)

Eine Lösung von 0,5 g (5E)-(16RS)-16-Methyl-3-oxa-18,18,19,19-tetradehydro-6a-carba-prostaglandin-$I_2$-11,15-bis-(tetrahydropyran-2-yläther) in 25 ml Methylenchlorid versetzt man unter Rühren bei 0°C tropfenweise mit einer ätherischen Diazomethanlösung bis zur bleibenden Gelbfärbung. Nach Abdampfen des Lösungsmittel reinigt man den Rückstand durch Chromatographie an Kieselgel mit Hexan/Äther (3 + 2) und erhält 0,45 g der Titelverbindung als Öl.

IR: 2945, 2870, 1750, 972/cm.

Beispiel 2

(5E)-2-Descarboxy-2-hydroxymethyl-3-oxa-16,16,20-trimethyl-18,18,19,19-tetrahydro-6a-carba-prostaglandin-$I_2$

In Analogie zu Beispiel 1 erhält man aus 280 mg des nach Beispiel 2e hergestellten Methylesters 268 mg (5E)-2-Descarboxy-2-hydroxymethyl-3-oxa-16,16,20-trimethyl-18,18,19,19-tetradehydro-6a-carba-prostaglandin-$I_2$-11,15-bis-(tetrahydropyranyläther) als farbloses Öl.

IR: 3600, 3430, 2940, 2870, 970/cm.

Nach Abspaltung der Schutzgruppen gemäß Beispiel 1 erhält man 120 mg der Titelverbindung als farbloses Öl

IR: 3610, 3430, 2925, 2865, 1600, 972/cm.

Das Ausgangsmaterial für die obige Titelverbindung wird wie folgt hergestellt:

2a) 3,3-Dimethyl-2-oxo-oct-5-inyl-phosphonsäure-dimethylester

Zu einer Suspension von 7,1 g Natriumhydrid (50%ige Suspension in Öl) in 220 ml abs. Tetrahydrofuran tropft man bei 24°C eine Lösung von 31,5 g 3-Methyl-2-oxo-butylphosphonsäuredimethylester in 74 ml abs. Tetrahydrofuran, rührt 1,5 Stunden und tropft anschließend bei 0°C 111 ml einer 1,6 molaren Butyllithiumlösung in Hexan zu und rührt 20 Minuten. In diese Lösung tropft man anschließend bei 0°C eine Lösung von 29 g 1-Brom-2-pentin in 44 ml abs. Tetrahydrofuran, rührt 3 Stunden bei 0°C, neutralisiert mit 3n Salzsäure und engt im Vakuum ein. Man versetzt mit 50 ml Sole, extrahiert dreimal mit je 200 ml Methylenchlorid, schüttelt den organischen Extrakt zweimal mit je 50 ml Sole, trocknet mit Magnesiumsulfat und dampft im Vakuum ein. Nach Destillation des Rückstandes bei 0,6 Torr und 125°C erhält man 23 g der Titelverbindung als farblose Flüssigkeit.

IR: 3000, 2962, 2860, 1720/cm.

2b) (1R,5S,6R,7R)-3,3-Äthylendioxy-7-benzoyloxy-6-[(E)-(3R)-3-hydroxy-4,4-dimethyl-non-1-en-6-inyl]-bicyclo[3.3.0]octan

Zu einer Suspension von 0,7 g Natriumhydrid (55%ige Suspension in Öl) in 60 ml Dimethoxyäthan (DME) tropft man bei 0°C eine Lösung aus 4,5 g des nach Beispiel 2a hergestellten Phosphonats in 35 ml DME und rührt 1 Stunde bei 0°C. Anschließend versetzt man bei −20°C mit einer Lösung aus 4,75 g (1R,5S,6R,7R)-3,3-Äthylendioxy-7-benzoyloxy-6-foryl-bicyclo[3.3.0]-octan in 60 ml DME, rührt 1,5 Stunden bei −20°C, gießt auf gesättigte Ammoniumchloridlösung und extrahiert mit Äther. Man wäscht den organischen Extrakt mit Wasser neutral, trocknet über Magnesiumsulfat und dampft im Vakuum ein. Nach Chromatographie des Rückstandes an Kieselgel erhält man mit Äther/Hexan (6 + 4) 4,7 g des ungesättigten Ketons als Öl.

IR: 2940, 2860, 1715, 1670, 1627, 948/cm.

Zu einer Lösung von 4,7 g des Ketons in 150 ml Methanol fügt man bei −40°C portionsweise 2,6 g Natriumborhydrid und rührt 1 Stunde bei −40°C. Anschließend verdünnt man mit Äther, wäscht mit Wasser neutral, trocknet über Magnesiumsulfat und dampft im Vakuum ein. Durch Säulenchromatographie an Kieselgel mit Äther/Hexan (7 + 3) erhält man zunächst 1,8 g der Titelverbindung (PG-Nomenklatur: 15α-Hydroxy) sowie als polarere Komponente 1,6 g der isomeren 15ß-Hydroxyverbindung als farblose Öle.

IR: 3610, 3410 (breit), 2943, 1712, 1603, 1588, 970, 948/cm.

2c) (IR,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-on

Eine Mischung aus 1,8 g des nach Beispiel 2b hergestellten α-Alkohols und 0,7 g Kaliumcarbonat in 60 ml Methanol rührt man 16 Stunden bei Raumtemperatur unter Argon. Anschließend engt man im Vakuum ein, verdünnt mit Äther und wäscht mit Sole neutural. Man trocknet über Magnesiumsulfat und dampft im Vakuum ein. Den Eindampfrückstand rührt man 16 Stunden bei Raumtemperatur mit 40 ml einer Mischung aus Essigsäure/Wasser/Tetrahydrofuran (65 + 35 + 10) und dampft anschließend im Vakuum ein. Nach Filtration des Rückstandes über Kieselgel erhält man mit Essigester/Hexan (7 + 3) 1,15 g des Ketons als Öl.

Eine Lösung aus 1,15 g des Ketons, 1,2 ml Dihydropyran und 10 mg p-Toluolsulfonsäure in 40 ml Methylenchlorid rührt man 30 Minuten bei 0°C. Anschließend verdünnt man mit Äther, schütelt mit verd. Natriumbicarbonat-Lösung, wäscht mit Wasser neutral, trocknet über Magnesiumsulfat und dampft im Vakuum ein. Man erhält 1,65 g des Bistetrahydropryanyläthers, der ohne weitere Reingung verwendet wird.

IR: 2962, 2865, 1738, 972/cm.

2d) 2-{(E)-(1S,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-äthan-1-ol

Zu einer Lösung von 2,1 g Phosphonessigsäuretriäthylester in 40 ml Tetrahydrofuran fügt man bei 0°C 0,9 g Kalium-tert.-butylat, rührt 10 Minuten, versetzt mit einer Lösung aus 2,2 g des nach Beispiel 2c hergestellten ketons in 20 ml Toluol und rührt 20 Stunden bei Raumtemperatur. Man verdünnt mit 200 ml Äther, schüttelt zweimal mit Wasser, einmal mit 20%iger Natronlauge, wäscht mit Wasser neutral, trocknet über Magnesiumsulfat und dampft in Vakuum ein. Den Rückstand filtriert man mit Hexan/Äther (3 + 2) über Kieselgel. Dabei erhält man 1,95 g des ungesättigten Esters als farbloses Öl.

IR: 2943, 2865, 1700, 1655, 972/cm.

Man fügt 0,6 g Lithiumaluminiumhydrid portionweise bei 0°C zu einer gerührten von 1,95 g des vorstehend hergestellten Esters in 60 ml Äther und rührt 30 Minuten bei 0°C. Man zerstört den Reagenzüberschuß durch tropfenweise Zugabe von Essigester, fügt 3 ml Wasser zu, rührt 2 Stunden bei 20°C, filtriert und dampft im Vakuum ein. Den Rückstand chromatographiert man mit Äther/Hexan (3 + 2) an Kieselgel. Dabei erhält man als unpolarere Verbindung 0,45 g 2-{(Z)-(1S,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-Äthan-1-ol und 0,7 g der Titelverbindung als farblose Öle.

IR: 3600, 3445, 2940, 2865, 1600, 972/cm.

2e) (5E)-3-Oxa-16,16,20-trimethyl-18,18,19,19-tetradehydro-6a-carba-prostaglandin-$I_2$-methylester-11,15-bis-(tetrahydropyranyl-äther)

Zu einer Lösung von 830 mg des nach Beispiel 2d hergestellten Alkohols in 13 ml Tetrahydrofuran fügt man 170 mg Natriumhydrid (55%ige Suspension in Öl) und rührt 30 Minuten bei 24°C. Anschließend tropft man eine Lösung von 270 mg Bromessigsäure in 4,4 ml Tetrahydrofuran zu und erhitz 24 Stunden am Rückfluß. Man kühlt ab, säuert mit 5%iger Schwefelsäure an, extrahiert mit Methylenchlorid, schüttelt mit Wasser und dampft im Vakuum ein. Den Rückstand nimmt man in 100 ml Äther auf und extrahiert viermal mit je 20 ml 4%iger Natronlauge. Man säuert die alkalische Phase mit 5%iger Schwefelsäure an, extrahiert mit Methylenchlorid, wäscht den organischen Extrakt dreimal mit Wasser, trocknet über Magnesiumsulfat und dampft in Vakuum ein. Dabei erhält man 640 mg der 3-Oxa-Säure als farbloses, dünnschichtchromatographisch einheitliches Öl, die man in Analogie zu Beispiel la in den Methylester überführt.

IR: 2945, 2865, 1748, 970/cm.

Beispiel 3

(5E)-2-Descarboxy-2-hydroxymethyl-3-oxa-16,16-dimethyl-18,18,19,19-tetradehydro-6a-carba-prostaglandin-$I_2$

In Analogie zu Beispiel 1 erhält man aus 550 mg des nach Beispiel 3d hergestellten Methylesters 540 mg (5E)-2-Descarboxy-2-hydroxymethyl-3-oxa-16,16-dimethyl-18,18,19,19-tetradehydro-6a-carba-prostaglandin-$I_2$-11,15-bis-(tetrahydropyranyläther) als farbloses Öl.

IR: 3600, 3450, 2942, 2865, 972/cm.

Nach Abspaltung der Schutzgruppen gamäß Beispiel 1 erhält man 280 mg der Titelvirbindung als farbloses Öl.

IR: 3600, 3450, 2926, 2865, 1600, 972/cm.

Das Ausgangsmaterial für die obige Titelverbindung wird wie folgt hergestellt:

3a) (1R,5S,6R,7R)-3,3-Äthylendioxy-7-benzoyloxy-6-[(E)-(3R)-3-hydroxy-4,4-dimethyl-oct-1-en-6-inyl]-bicyclo[3.3.0]-octan

In Analogie zu Beispiel 2b erhält man aus 9,4 g (1R,5S,6R,7R)-3,3-Äthylendioxy-7-benzoyloxy-6-formyl-bicyclo[3.3.0]-octan und 9,1 g 3,3-Dimethyl-2-oxo-oct-5-inyl-phosponsäure-dimethylester 9,2 g des ungesättigten Ketons. Durch Reduktion mit Natriumborhydrid erhält man 3,7 g der Titelverbindung als farbloses Öl.

IR: 3600, 3400, 2942, 1712, 1602, 1589, 972, 949/cm.

3b) (1R,5S,6R,7R)-7-(Tetrahydropyran-2-yloxy)-6-[(E)-(3R)-4,4-dimethyl-3-(tetrahydropyran-2-yloxy)-oct-1-en-6-inyl]-bicyclo[3.3.0]octan-3-on

In Analgoie zu Beispiel 2c erhält man aus 3,7 g des nach Beispiel 3a hergestellten α-Alkohols 3,4 g des Bistetrahydropyranyläthers als farbloses Ö1.

IR: 2960, 2865, 1737, 970/cm.

3c) 2-{(E)-(1S,5S,6R,7R)-7-(Tetrahydropyran-2-yloxy)-6-[(E)-(3R)-4,4-dimethyl-3-(tetrahydropyran-2-yloxy)-oct-1-en-6-inyl]-bicyclo[3.3.0]octan-3-yliden}-äthan-1-ol

In Analogie zu Beispiel 2d erhält man aus 3,2 g des nach Beispiel 3b hergestellten Ketons 1,1 g der Titelverbindung als farbloses Öl.

IR: 3610, 3440, 2942, 2865, 1600, 970/cm.

3d) (5E)-16,16-Dimethyl-3-oxa-18,18,19,19-tetradehydro-6a-carba-prostaglandin-$I_2$-methylester-11,15-bis-(tetrahydropyranyläther)

In Analogie zu Beispiel 2e erhält man aus 1 g des nach Beispiel 3c hergestellten Alkohols 760 mg der Titelverbindung als farbloses Öl.

IR: 2945, 2865, 1750, 972/cm.

Biespiel 4

(5Z)-(16RS)-2-Descarboxy-2-hydroxymethyl-3-oxa-16-methyl-18,18,19,19-tetradehydro-6a-carba-prostaglandin-$I_2$

In Analogie zu Beispiel 1 erhält man aus 250 mg (5Z)-(16RS)-16-Methyl-3-oxa-18,18,19,19-tetradehydro-6a-carba-prostaglandin-$I_2$-methylester-11,15-bis(tetrahydropyranyläther) (hergestellt aus der entsprechenden Säure gemäß Beispiel 1b) 240 mg (5Z)-(16RS)-2-Descarboxy-2-hydroxymethyl-3-oxa-16-methyl-18,18,19,19-tetradehydro-6a-carba-prostaglandin-$I_2$-11,15-bis-(tetradehydropyranyläther) als farbloses Öl.

IR: 3610, 3450, 2943, 2865, 1453, 1440, 970/cm.

Nach Abspaltung der Schutzgruppen gemäß Beispiel 1 erhält man 165 mg der Titelverbindung als farbloses Öl.

IR: 3600, 3435, 2922, 2860, 1600, 1456, 1430, 1105, 970/cm.

Beispiel 5

(5E)-(16RS)-2-Descarboxy-16,20-dimethyl-2-hydroxymethyl-3-oxa-18,18,19,19-tetradehydro-6a-carba-prostaglandin-$I_2$

In Analogie zu Beispiel 1 erhält man aus 340 mg (5E)-(16RS)-16,20-dimethyl-3-oxa-18,18,19,19-tetrahydro-6-carba-prostaglandin-$I_2$-methylester-11,15-bis-(terahydropyranyläther) (hergestellt aus der entsprechenden Säure mit Diazomethan gemäß Beispiel 1a) 335 mg (5E)-(16RS)-2-Descarboxy-16,20-dimethyl-2-hydroxymethyl-3-oxa-18,18,19,19-tetradehydro-6a-carba-prostaglandin-$I_2$-11,15-bis-(tetrahydropyranyl-äther) als farbloses Öl.

IR: 3600, 3400, 2942, 2870, 972/cm.

Nach Abspaltung der Schutzgruppen gemäß Beispiel 1 erhält man 145 mg der Titelverbindung als Öl.

IR: 3610, 3450, 2925, 2860, 1600, 9762/cm.

Beispiel 6

(5E)-(16RS)-2-Descarboxy-16,20-dimethyl-2-hydroxymethyl-3-oxa-19,19,20,20-tetradehydro-6a-carba-prostaglandin-$I_2$

In Analogie zu Beispiel 1 erhält man aus 390 mg (5E)(16RS)-16,20-dimethyl-3-oxa-19,19,20,20-tetradehydro-6a-carba-prostaglandin-$I_2$-methylester-11,15-bis-(tetrahydropyranäther) (hergestellt aus der entsprechenden Säure mit Diazomethan gemäß Beispiel 1a) 385 mg (5E)-(16RS)-2-Descarboxy-16,20-dimethyl-2-hydroxymethyl-3-oxa-19,19,20,20-tetradehydro-6a-carba-prostaglandin-$I_2$ -11,15-bis-(tetrahydropyranyläther) als farbloses Öl.

IR: 3610, 3450, 2940, 2870, 1602, 974/cm.

Nach Abspaltung der Schutzgruppen gemäß Beispiel 1 erhält man 220 mg der Titelverbindung als farbloses Öl.

IR: 3610, 3450, 2925, 2862, 1600, 1455, 1432, 1105, 974/cm.

Beispiel 7

(5E)-2-Descarboxy-2-hydroxymethyl-3-oxa-18,18,19,19-tetradehydro-6a-carba-prostaglandin-$I_2$

In Analogie zu Beispiel 1 erhält man aus 540 mg (5E)-3-oxa-18,18,19,19-tetradehydro-6a-carba-prostaglandin-$I_2$-methylester-11,15-bis-(tetrahydropyranyläther) (hergestellt aus der entsprechenden Säure mit Diazomethan gemäß Beispiel 1a) 530 mg (5E)-2-Descarboxy-2-hydroxymethyl-3-oxa-18,18,19,19-tetradehydro-6a-carba-prostaglandin-$I_2$-11,15-bis-(tetrahydropyranyläther) als farbloses Öl.

IR: 3600, 3420, 2942, 2865, 970/cm.

Nach Abspaltung der Schutzgruppen gemäß Beispiel 1 erhält man 290 mg der Titelverbindung als Öl.

IR: 3610, 3440, 2925, 2862, 1602, 1455, 1432, 1105, 972/cm.

Beispiel 8

(5E)-(16RS)-2-Descarboxy-16,19-dimethyl-2-hydroxymethyl-3-oxa-18,19-didehydro-6a-carba-prostaglandin-$I_2$

In Analogie zu Beispiel erhält man aus 290 mg (5E)-(16RS)-16,19-dimethyl-3-oxa-18,19-didehyro-6a-carba-prostaglandin-$I_2$-methylester-11,15-bis-(tetrahydropyranyläther) (hergestellt aus der entsprechenden Säure gemäß Beispiel 1) 280 mg (5E)-(16RS)-2-Descarboxy-16,19-dimethyl-2-hydroxymethyl-3-oxa-18,19-didehydro-6a-carba-prostaglandin-$I_2$-11,15-bis-(tetrahydropyranyläther) als farbloses Öl.

IR: 3600, 3400, 2945, 2865, 974/cm.

Nach Abspaltung der Schutzgruppen gemäß Beispiel 1 erhält man 155 mg der Titelverbindung als Öl.

IR: 3600, 3400, 2925, 2865, 1600, 1455, 1435, 1105, 974/cm.

Beispiel 9

(5E)-(16RS)-2-Descarboxy-2-hydroxymethyl-16-methyl-18,18,19,19-tetradehydro-6a-carba-prostaglandin-$I_2$

In Analogie zu Beispiel 1 erhält man aus 400 mg (5E)-(16RS)-16-methyl-18,18,19,19-tetradehydro-6a-carba-prostaglandin-$I_2$-methylester-11,15-bis-(tetrahydropyranyläther) (hergestellt aus der entsprechenden Säure mit Diazomethan gemäß Beispiel 1a) 385 mg (5E)-(16RS)-2-Descarboxy-2-hydroxymethyl-16-methyl-18,18,19,19-tetradehydro-6a-carba-prostaglandin-$I_2$-11,15-bis-(tetrahydropyranyläther) als farbloses Öl.

IR: 3610, 3450, 2945, 2865, 974/cm.

Nach Abspaltung der Schutzgruppe gemäß Beispiel 1 erhält man 235 mg der Titelverbindung als farbloses Öl.

IR: 3600, 3400, 2925, 2865, 1600, 974/cm.

Beispiel 10

(5E)-2-Descarboxy-2-hydroxymethyl-16,16-20-trimethyl-18,18,19,19-tetradehydro-6a-carba-prostaglandin-$I_2$

In Analogie zu Beispiel 1 erhält man aus 550 mg des nach Beispiel 10a hergestellten Methylesters 540 mg (5E)-2-Descarboxy-2-hydroxymethyl-16,16,20-trimethyl-18,18,19,19-tetradehydro-6a-carba-prostaglandin-$I_2$-11,15-bis-(tetrahydropyranyläther) als farbloses Öl.

IR: 3600, 3420, 2940, 2865, 972/cm.

Nach Abspaltung der Schutzgruppen gemäß Beispiel 1 erhält man 325 mg der Titelverbindung als farbloses Ol.

IR: 3600, 3400, 2925, 2865, 1600, 972/cm.

Das Ausgangsmaterial für die obige Titelverbindung wird wie folgt hergestellt:

10a) (5E)-16,16,20-Trimethyl-18,18,19,19-tetradehydro-6a-carba-prostaglandin-$I_2$-methylester-11,15-bis-(tetrahydropyranyläther)

Zu einer Lösung von 15 g 4-Carboxybutyltriphenylphosphoniumbromid in 35 ml abs. Dimethylsulfoxid (DMSO) tropft man bei 15°C unter Argon 60 ml einer 1,04 molaren Lösung von Methylsulfinylmethylnatrium in DMSO und rührt 30 Minuten bei 20°C. Zur roten Ylenlösung tropft man eine Lösung von 2,5 g des nach Beispiel 2c hergestellten Ketons in 15 ml abs. DMSO un rührt 5 Stunden bei 45°C. Das Reaktionsgemisch wird auf Eiswasser gegossen, mit 10%iger Zitronensäurelösung auf pH 4—5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstands an Kieselgel erhält man mit Äther/Hexan (1 + 1) zunächst 0,7 g (5Z)-16,16,20-trimethyl-18,18,19,19-tetradehydro-6a-carba-

prostaglandin-$I_2$-11,15-bis-(tetrahydropyran-2-yläther) sowie als polarere Komponente 1,2 g (5E)-16,16,20-trimethyl-18,18,19,19-tetradehydro-6a-carba-prostaglandin-$I_2$-11,15-bis-(tetrahydropyranyläther) als farblose Öle.

IR: 3510 (breit), 2960, 2868, 1710, 974/cm.

Zur Veresterung löst man 1,2 g der vorstehend erhaltenen (E)-konfigurierten Säure in 60 ml Methylenchlorid und versetzt bei 0°C tropfenweise mit einer ätherischen Diazomethanlösung bis zur bleibenden Gelbfärbung. Nach Abdampfen des Lösungsmittels reinigt man den Rückstand durch Chromatographie an Kieselgel mit Hexan/Äther (3 + 2) und erhält 1,1 g der Titelverbindung als Öl.

IR: 1735, 875/cm.

## Beispiel 11

(5E)-2-Descarboxy-16,16-dimethyl-2-hydroxymethyl-18,18,19,19-tetradehydro-6a-carba-prostaglandin-$I_2$

In Analogie zu Beispiel 1 erhält man aus 320 mg des nach Beispiel 12a hergestellten Methylesters 305 mg (5E)-2-Descarboxy-16,16-20-dimethyl-2-hydroxymethyl-18,18,19,19-tetradehydro-6a-carba-prostaglandin-$I_2$-11,15-bis-(tetrahydropyranyläther) als farbloses Öl.

IR: 3610, 3400, 2942, 2866, 974/cm.

Nach Abspaltung der Schutzgruppen gemäß Beispiel 1 erhält man 170 mg der Titelverbindung als Öl.

IR: 3600, 3450, 2928, 2865, 1602, 974/cm.

Das Ausgangsmaterial für die obige Titelverbindung wird wie folgt hergestellt:

11a) (5E)-16,16-Dimethyl-18,18,19,19-tetradehydro-6a-carba-prostaglandin-$I_2$-methylester-11,15-bis-(tetrahydropyranyläther)

In Analogie zu Beispiel 10a erhält man aus 2,8 g des nach Beispiel 3b hergestellten Ketons 1,35 g (5E)-16,16-Dimethyl-18,18,19,19-tetradehydro-6a-carba-prostaglandin-$I_2$-11,15-bis-(tetrahydropyranyläther) als farbloses Öl.

IR: 3500 (breit), 2962, 2870, 1712, 975/cm.

Durch Veresterung der vorstehend erhaltenen Säure mit ätherischer Diazomethanlösung gemäß Beispiel 10a erhält man 1,2 g der Titelverbindung als Öl.

IR: 1736, 975/cm.

## Beispiel 12

(5E)-(16RS)-2-Descarboxy-16,20-dimethyl-2-hydroxymethyl-18,18,19,19-tetradehydro-6a-carba-prostaglandin-$I_2$

In Analogie zu Beispiel 1 erhält man aus 420 mg (5E)-(16RS)-16,20-dimethyl-18,18,19,19-tetradehydro-6a-carba-prostaglandin-$I_2$-methylester-11,15-bis-(tetrahydropyranyläther) (hergestellt aus der entsprechenden Säure mit Diazomethan gemäß Beispiel 1) 400 mg (5E)-(16RS)-2-Descarboxy-16,20-dimethyl-2-hydroxymethyl-18,18,19,19-tetradehydro-6a-carba-prostaglandin-$I_2$-11,15-bis-(tetrahydropyranyläther) als farbloses Öl.

IR: 3610, 3450, 2945, 2865, 975/cm.

Nach Abspaltung der Schutzgruppen gemäß Beispiel 1 erhält man 270 mg der Titelverbindung als Öl.

IR: 3600, 3400, 2930, 2860, 1602, 975/cm.

## Beispiel 13

(5E)-(16RS)-2-Descarboxy-16,20-dimethyl-2-hydroxymethyl-19,19,20,20-tetradehydro-6a-carba-prostaglandin-$I_2$

In Analogie zu Beispiel 1 erhält man aus 400 mg (5E)-(16RS)-16,20-Dimethyl-19,19,20,20-tetradehydro-6a-carba-prostaglandin-$I_2$-methylester-11,15-bis-(tetrahydropyranyläther) (hergestellt aus der entsprechenden Säure mit Diazomethan gemäß Beispiel 1) 370 mg (5E)-(16RS)-2-Descarboxy-16,20-dimethyl-2-hydroxymethyl - 19,19,20,20 - tetradehydro - 6a - carba - prostaglandin - $I_2$ - 11,15 - bis - (tetrahydropyranyläther) als farbloses Öl.

IR: 3600, 3450, 2950, 2862, 976/cm.

Die Abspaltung der Schutzgruppen erfolgt analog Beispiel 1. Man erhält 200 mg der Titelverbindung als Öl.

IR: 3600, 3420, 2944, 2860, 1600, 976/cm.

## Beispiel 14

(5E)-2-Descarboxy-2-hydroxymethyl-18,18,19,19-tetradehydro-6a-carba-prostaglandin-$I_2$

In Analogie zu Beispiel 1 erhält man aus 400 mg (5E)-18,18,19,19-tetradehydro-6a-carba-prostaglandin-$I_2$-methylester-11,15-bis-(tetrahydropyranyläther) (hergestellt aus der entsprechenden Säure und Diazomethan gemäß Beispiel 1) 365 mg (5E)-2-Descarboxy-2-hydromethyl-18,18,19,19-tetradehydro-6a-carba-prostaglandin-$I_2$-11,15-bis-(tetrahydropyranyläther) als Öl.

IR: 3620, 3450, 2952, 2866, 978/cm.

Die Abspaltung der Schutzgruppen erfolgt analog Beispiel 1. Man erhält 185 mg der Titelverbindung als Öl.

IR: 3600, 3425, 2948, 2860, 978/cm.

Beispiel 15

(5E)-(16RS)-2-Descarboxy-16,19-dimethyl-18,19-didehdro-2-hydroxymethyl-6a-carba-prostaglandin-$I_2$

In Analogie zu Beispiel 1 erhält man aus 500 mg (5E)-(16RS)-16,19-Dimethyl-18,19-didehydro-6a-carba-prostaglandin-$I_2$-methylester-11,15-bis-(tetrahydropyranyläther) (hergestellt aus der entsprechenden Säure mit Diazomethan gemäß Beispiel 1) 430 mg (5E)-(16RS)-2-Descarboxy-16,19-dimethyl-18,19-didehydro-2-hydroxymethyl-6a-carba-prostaglandin-$I_2$-11,15-bis-(tetrahydropyranyläther) als Öl.

IR: 3610, 3452, 2950, 2864, 980/cm.

Die Abspaltung der Schutzgruppen erfolgt analog Beispiel 1. Man erhält 250 mg Titelverbindung als Öl.

IR: 3600, 3420, 2946, 2858, 1602, 978/cm.

**Patentansprüche**

1. Carbacyclinderivate der allgemeinen Formel I

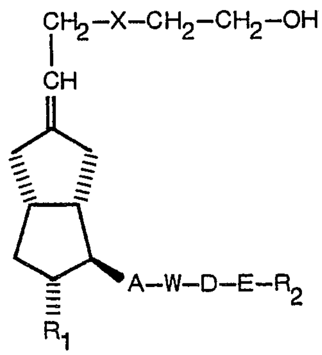

I,

worin

X eine Sauerstoffatom oder eine —$CH_2$-Gruppe,

A eine —$CH_2$—$CH_2$—, eine trans-CH=CH— oder eine —C≡C-Gruppe,

W eine freie oder funktionell abgewandelte Hydroxymethylen-gruppe, wobei die OH-Gruppe α- oder β-ständig sein kann,

D eine geradkettige, gesättigte Alkylengruppe mit 1—10 C-Atomen oder eine verzweigte gesättigte oder ungesättigte Alkylengruppe mit 2—10 C-Atomen, die durch $C_1$—$C_4$-Alkyl oder Fluoratome substituiert sein können und Doppelbindungen sich in 2- oder 3-Stellung befinden, wenn D 4—10 C-Atome hat,

E eine —C≡C-Gruppe oder eine —$CR_3$=$CR_4$-Gruppe, wobei $R_3$ und $R_4$ verschieden sind und ein Wasserstoffatom oder eine Alkylgruppe mit 1—5 C-Atomen bedeuten,

$R_1$ eine freie oder funktionell abgewandelte Hydroxygruppe,

$R_2$ gerad- oder verzweigtkettiges, gesättigtes oder ungesättigtes Alkyl 1—10 C-Atomen, Cycloalkyl mit 4—10 C-Atomen, Phenyl, 1-Naphthyl und 2-Naphthyl, durch 1—3 Halogenatome, eine Phenylgruppe, 1—3 Alkylgruppen mit jeweils 1—4 C-Atomen, eine Chlormethyl, Fluormethyl, Trifluormethyl, Carboxyl oder $C_1$—$C_4$-Alkoxy- oder Hydroxygruppe substituiertes Phenyl, 1-Naphthyl oder 2-Naphthyl, oder eine 5- oder 6-gliedrige heterocyclische Gruppe mit einem N-, O- oder S-Atom bedeuten.

2. Verfahren zur Herstellung von Carbacyclinderivaten der allgemeinen Formel I, dadurch gekennzeichnet, daß man in an sich bekannter Weise eine Verbindung de allgemeinen Formel II

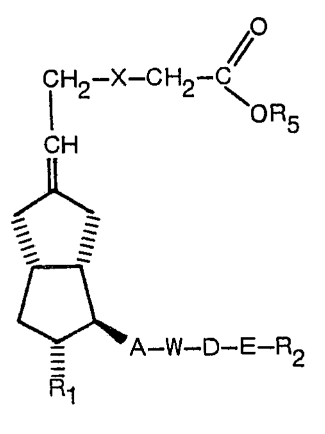

II,

worin

$R_1$, $R_2$, X, A, W, D und E die obenangegebenen Bedeutungen aufweisen und $R_5$ einen Alkylrest mit 1—5 C-Atomen oder ein Waserstoffatom bedeutet, reduziert und gegebenenfalls anschließend in beliebiger

Reihenfolge Isomere trennt und/oder geschützte Hydroxygruppen freisetzt.

3. Arzneimittel, bestehend aus einer oder mehreren Verbindungen gemäß Anspruch 1 und üblichen Hilfs- und Trägerstoffen.

4. (5E)-(16RS)-2-Descarboxy-2-hydroxymethyl-3-oxa-16-methyl-18,18,19,19-tetradehydro-6a-carba-prostaglandin-$I_2$.

5. (5E)-2-Descarboxy-2-hydroxymethyl-3-oxa-16,16,20-trimethyl-18,18,19,19-tetradehydro-6a-carba-prostaglandin-$I_2$.

6. (5E)-2-Descarboxy-2-hydroxymethyl-3-oxa-16,16-dimethyl-18,18,19,19-tetradehydro-6a-carba-prostaglandin-$I_2$.

7. (5Z)-(16RS)-2-Descarboxy-2-hydroxymethyl-3-oxa-16-methyl-18,18,19,19-tetradehydro-6a-carba-prostaglandin-$I_2$.

8. (5E)-(16RS)-2-Descarboxy-16,20-dimethyl-2-hydroxymethyl-3-oxa-18,18,19,19-tetradehydro-6a-carba-prostaglandin-$I_2$.

9. (5E)-(16RS)-2-Descarboxy-16,20-dimethyl-2-hydroxymethyl-3-oxa-19,19,20,20-tetradehydro-6a-carba-prostaglandin-$I_2$.

10. (5E)-2-Descarboxy-2-hydroxymethyl-3-oxa-18,18,19,19-tetradehydro-6a-carba-prostaglandin-$I_2$.

11. (5E)-(16RS)-2-Descarboxy-16,19-dimethyl-2-hydroxymethyl-3-oxa-18,19-didehydro-6a-carba-prostagladin-$I_2$.

12. (5E)-(16RS)-2-Descarboxy-2-hydroxymethyl-16-methyl-18,18,19,19-tetradehydro-6a-carba-prostaglandin-$I_2$.

13. (5E)-2-Descarboxy-2-hydroxymethyl-16,16-20-trimethyl-18,18,19,19-tetradehydro-6a-carba-prostaglandin-$I_2$.

14. (5E)-2-Descarboxy-16,16-dimethyl-2-hydroxymethyl-18,18,19,19-tetradehydro-6a-carba-prostaglandin-$I_2$.

15. (5E)-(16RS)-2-Descarboxy-16,20-dimethyl-2-hydroxymethyl-18,18,19,19-tetradehydro-6a-carba-prostaglandin-$I_2$.

16. (5E)-(16RS)-2-Descarboxy-16,20-dimethyl-2-hydroxymethyl-19,19,20,20-tetradehydro-6a-carba-prostaglandin-$I_2$.

17. (5E)-2-Descarboxy-2-hydroxymethyl-18,18,19,19-tetradehydro-6a-carba-prostaglandin-$I_2$.

18. (5E)-(16RS)-2-Descarboxy-16,19-dimethyl-18,19-didehydro-2-hydoxymethyl-6a-carba-prostaglandin-$I_2$.

**Revendications**

1. Carbacyclines répondant à la formule générale I:

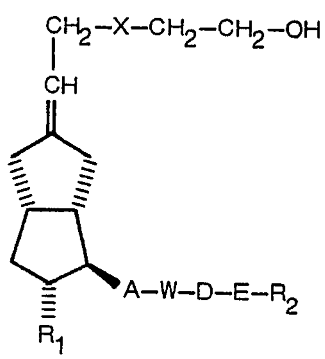

I,

dans laquelle:

X représente un atome d'oxygène ou un radical —$CH_2$—,

A représente un radical —$CH_2$—$CH_2$—, un radical —CH=CH— trans ou un radical —C≡C—,

W représente un radical hydroxyméthylène, dont le radical —OH peut avoir la configuration α ou la configuration β, et peut être libre ou sous la forme d'un dérivé fonctionnel,

D représente un radical alkylène linéaire saturé contennant de 1 à 10 atomes de carbone on un radical alkylène ramifié, saturé ou insaturé, contenant de 2 à 10 atomes de carbone, qui peut porter un alkyle en $C_1$—$C_4$ ou un atome de fluor, l'alkylène insaturé contenant une double liaison en position 2 ou en position 3 dans le cas où D contient de 4 à 10 atomes de carbone,

E représente un radical —C≡C— ou un radical —$CR_3$=$CR_4$— dans lequel $R_3$ et $R_4$ sont différents l'une de l'autre et représentent chacun un atome d'hydrogène ou un alkyle contenant de 1 à 5 atomes de carbone,

$R_1$ représente un radical hydroxy libre ou sous la forme d'un dérivé fonctionnel,

$R_2$ représente un radical alkyle linéaire ou ramifié, saturé ou insaturé, qui contient de l à 10 atomes de carbone, un radical cycloalkyle contenant de 4 à 10 atomes de carbone, un radical phényle, un radical

naphtyle-1 ou naphtyle-2, un radical phényle, naphtyle-1 ou naphtyle-2 porteur de 1 à 3 atomes d'halogène, d'un phényle, de 1 à 3 alkyles en $C_1$—$C_4$, d'un chlorométhyle, d'un fluorométhyle, d'un trifluorométhyle, d'un carboxy, d'un alcoxy en $C_1$—$C_4$ ou d'un hydroxy, ou un radical hétérocyclique à 5 ou 6 maillons qui contient un atome N, O ou S.

2. Procédé de préparation de carbacyclines de formule générale I, procédé caractérisé en ce qu'on réduit, de manière connue, un composé répondant à la formule générale II:

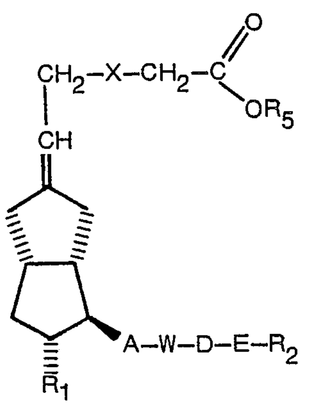

II,

dans laquelle $R_1$, $R_2$, X, A, W, D et E ont les significations précédemment données et $R_5$ représente un radical alkyle contenant de 1 à 5 atomes de carbone ou un atome d'hydrogène, puis, le cas échéant, en opérant dans l'ordre que l'on veut, on sépare des isomères et/ou on libère des radicaux hydroxy protégés.

3. Médicament constitué d'un ou de plusieurs composés selon la revendication 1 et d'adjuvants et excipients usuels.

4. Décarboxy-2 hydroxyméthyl-2 oxa-3 méthyl-16 tétradéhydro-18,18,19,19 carba-6a prostaglandine-$I_2$-(5E)-(16RS).

5. Décarboxy-2 hydroxyméthyl-2 oxa-3 triméthyl-16,16,20 tétradéhydro-18,18,19,19 carba-6a prostaglandine-$I_2$-(5E).

6. Décarboxy-2 hydroxyméthyl-2 oxa-3 diméthyl-16,16 tétradéhydro-18,18,19,19 carba-6a prostaglandine-$I_2$-(5E).

7. Décarboxy-2 hydroxyméthyl-2 oxa-3 méthyl-16 tétradéhydro-18,18,19,19 carba-6a prostaglandine-$I_2$-(5Z)-(16RS).

8. Décarboxy-2 diméthyl-16,20 hydroxyméthyl-2 oxa-3 tétradéhydro-18,18,19,19 carba-6a prostaglandine-$I_2$-(5E)-(16RS).

9. Décarboxy-2 diméthyl-16,20 hydroxméthyl-2 oxa-3 tétradéhydro-19,19,20,20 carba-6a prostaglandine-$I_2$-(5E)-(16RS).

10. Décarboxy-2 hydroxyméthyl-2 oxa-3 tétradéhydro-18,18,19,19 carba-6a prostaglandine-$I_2$-(5E).

11. Décarboxy-2 diméthyl-16,19 hydroxyméthyl-2 oxa-3 didéhydro-18,19 carba-6a prostaglandine-$I_2$-(5E)-(16RS).

12. Décarboxy-2 hydroxyméthyl-2 méthyl-16 tétradéhydro-18,18,19,19 carba-6a prostaglandine-$I_2$-(5E)-(16RS).

13. Décarboxy-2 hydroxyméthyl-2 triméthyl-16,16,20 tétradéhydro-18,18,19,19 carba-6a prostaglandine-$I_2$-(5E).

14. Décarboxy-2 diméthyl-16,16 hydroxyméthyl-2 tétradéhydro-18,18,19,19 carba-6a prostaglandine-$I_2$-(5E).

15. Décarboxy-2 diméthyl-16,20 hydroxyméthyl-2 tétradéhydro-18,18,19,19 carba-6a prostaglandine-$I_2$-(5E)-(16RS).

16. Décarboxy-2 diméthyl-16,20 hydroxyméthyl-2 tétradéhydro-19,19,20,20 carba-6a prostaglandine-$I_2$-(5E)-(16RS).

17. Décarboxy-2 hydroxyméthyl-2 tétradéhydro-18,18,19,19 carba-6a prostaglandine-$I_2$-(5E).

18. Décarboxy-2 diméthyl-16,19, didéhydrotétradéhydro-18,19 hydroxyméthyl-2 carba-6a prostaglandine-$I_2$-(5E)-(16RS).

**Claims**

1. Carbacyclin derivatives of the general formula I:

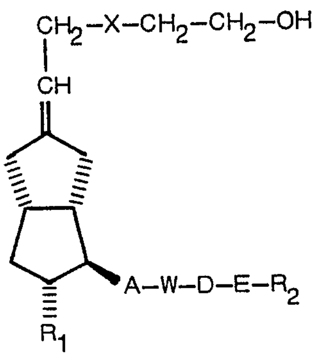

I,

wherein

X is an oxygen atom or a $CH_2$ group.

A is a $—CH_2—CH_2—$, trans—CH=CH— or —C≡C— group

W is a free or functionally modified hydroxymethylene group, where the OH group may be in the α- or β-configuration,

D is a straight-chain, saturated alkylene group of 1 to 10 C-atoms or a branched, saturated or unsaturated alkylene group of 2 to 10 C-atoms which can be substituted by $C_1$—$C_4$-alkyl of fluorine atoms, and when D has 4—10 atoms, double bonds are in the 2- or 3-position,

E is a —C≡C— group or a $—CR_3{=}CR_4—$ group, where $R_3$ and $R_4$ are different and are a hydrogen atom or an alkyl group of 1 to 5 C-atoms,

$R_1$ is a free or functionally modified hydroxy group,

$R_2$ is a straight or branched chain, saturated or unsaturated alkyl of 1 to 10 C-atoms, cycloalkyl of 4 to 10 C-atoms, phenyl, 1-naphthyl or 2-naphthyl, or phenyl, 1-naphthyl or 2-naphthyl, which can each be substituted by 1—3 halogen atoms, a phenyl group, 1—3 alkyl groups of 1—4 C-atoms, a chloromethyl, fluoromethyl, carboxy or $C_1$—$C_4$-alkoxy- or hydroxy group, or is a 5 or 6 membered heterocyclic group with an N-, O- or S-atom.

2. Process for the preparation of carbacyclin derivatives of general formula I, characterised in that, in known manner, a compound of the general formula II

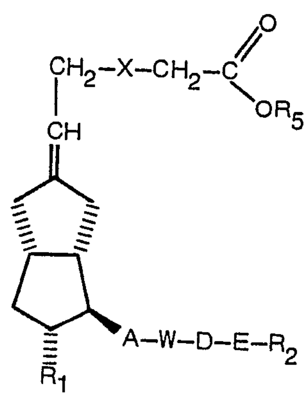

II,

wherein $R_1$, $R_2$, X, A, W, D and E have the meanings given above and $R_5$ is an alkyl group of 1—5 C-atoms or a hydrogen atom, is reduced and optionally subsequently and in any order, isomers are separated and/or protected hydroxy groups are freed.

3. Pharmaceuticals containing one or more compounds according to claim 1 and conventional additives and carriers.

4. (5E)-(16RS)-2-Descarboxy-2-hydroxymethyl-3-oxa-16-methyl-18,18,19,19-tetradehydro-6a-carba-prostaglandin-$I_2$.

5.(5E)-2-Descarboxy-2-hydroxymethyl-3-oxa-16,16,20-trimethyl-18,18,19,19-tetradehydro-6a-carba-prostaglandin-$I_2$.

6. (5E)-2-Descarboxy-2-hydroxymethyl-3-oxa-16,16-dimethyl-18,18,19,19-tetradehydro-6a-carba-prostaglandin-$I_2$.

7. (5Z)-(16RS)-2-Descarboxy-2-hydroxymethyl-3-oxa-16-methyl-18,18,19,19-tetradehydro-6a-carbaprostaglandin-$I_2$.
8. (5E)-(16RS)-2-Descarboxy-16,20-dimethyl-2-hydroxymethyl-3-oxa-18,18,19,19-tetradehydro-6a-carbaprostaglandin-$I_2$.
9. (5E)-(16RS)-2-Descarboxy-16,20-dimethyl-2-hydroxymethyl-3-oxa-19,19,20,20-tetradehydro-6a-carbaprostaglandin-$I_2$.
10. (5E)-2-Descarboxy-2-hydroxymethyl-3-oxa-18,18,19,19-tetradehydro-6a-carbaprostaglandin-$I_2$.
11. (5E)-(16RS)-2-Descarboxy-16,19-dimethyl-2-hydroxymethyl-3-oxa-18,19-didehydro-6a-carbaprostaglandin-$I_2$.
12. (5E)-(16RS)-2-Descarboxy-2-hydroxymethyl-16-methyl-18,18,19,19-tetradehydro-6a-carbaprostaglandin-$I_2$.
13. (5E)-2-Descarboxy-2-hydroxymethyl-16,16,20-trimethyl-18,18,19,19-tetradehydro-6a-carbaprostaglandin-$I_2$.
14. (5E)-2-Descarboxy-16,16-dimethyl-2-hydroxymethyl-18,18,19,19-tetradehydro-6a-carbaprostaglandin-$I_2$.
15. (5E)-(16RS)-2-Descarboxy-16,20-dimethyl-2-hydroxymethyl-18,18,19,19-tetradehydro-6a-carbaprostaglandin-$I_2$.
16. (5E)-(16RS)-2-Descarboxy-16,20-dimethyl-2-hydroxymethyl-19,19,20,20-tetradehydro-6a-carbaprostaglandin-$I_2$.
17. (5E)-2-Descarboxy-2-hydroxymethyl-18,18,19,19-tetradehydro-6a-carbaprostaglandin-$I_2$.
18. (5E)-(16RS)-2-Descarboxy-16,19-dimethyl-18,19-didehydro-2-hydroxymethyl-6a-carbaprostaglandin-$I_2$.